# EUROPEAN PATENT APPLICATION

(11) **EP 2 020 238 A1**
(43) Date of publication of application: **04.02.2009**
(21) Application number: 08012199.9
(22) Date of filing: 07.07.2008
(51) Int. Cl.: A61K 36/28

(54) **Mechanically treated milk thistle fruit and a method for its treatment**

(30) Priority: 02.08.2007 CZ 20070515
(71) Applicant: IREL, spol. s.r.o., 638 00 Brno-Lesna (CZ)
(72) Inventor: Buchta, Martin, 747 14 Ludgerovice (CZ); Svehlik, Zdenek, 671 72 Miroslav (CZ)
(74) Representative: Andera, Jiri

(57) **Abstract**

The invention relates to the mechanically treated milk thistle fruit and a method of its treatment for its subsequent extraction, or for direct use in the food or feeding industry, when the incoming fruit is first provided with pre-drying if necessary, then the fruit is subject to separation of mechanical impurities if necessary while after that there is the stage of removing the surface layers of the fruit with optionally separation of the fruit and subsequent sorting into the fraction of fruit coats and the fraction of the remaining inner layers of the fruit while all the materials can be preferably further processed by pressing to eliminate the fatty fraction, increase the content of flavonolignans and obtain the final structure when the shape and size of individual final products is achieved through crushing and sieving.

## Description

### Technical Field

The invention relates to a mechanically treated milk thistle fruit (Silybum marianum (L.) Gaertner) (hereinafter the fruit only, generally called the drug) and a method of its treatment, which is used as a source of biologically active substances, in particular flavonolignans (silymarin) for human and veterinary use.

### Background Art

The milk thistle fruits contain a number of substances out of which the most significant ones from the pharmacological point of view are substances of the flavonol-lignan type generally referred to as flavonolignans or also flavolignans. For their hepatoprotective, antioxidation, galactogenic and favourable vascular effects these substances are used in the state of the art as efficient phyto-pharmaceuticals both in classical (see e.g. the published International Application No. WO 2000/078325, European Patent No. EP 1 774 963 and the Russian Patents RU 2 291 706 and RU 2 274 400) and in alternative medicine (see e.g. the Czech Patents No. CZ 293 046 and CZ 296 773 and the published Czech Application No. PV 2004-905) and in cosmetic agents (see e.g. the published Japanese Application No. JP 2006282568 and the published German Application No. DE 102005063063). However, according to the state of the art their use is not only limited to the human sphere, but in the form of feeding mixtures or charges they are also administered to farm animals, especially cattle and poultry with the aim to improve efficiency of these animals, i.e. to increase the quality of their products as mentioned in literature, e.g. Vojtí 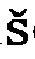 ek B. et al.: *Veterinární medicína (Veterinary Medicine)*, 321-330, 1991; Tedesco D. et al.: J. Dairy Sci. 87(7), 2239-2247, 2004; Tedesco D. et. al.: J. Vet. Med. A Physiol. Pathol. Clin. Med.. 51(2), 85-89, 2004; Chon S. K. and Kim N. S.: Parasitol. Res. 97(6), 445-451, 2005; and further the French Patent No. FR 2 719 451 A1, (Czech Patent No. CZ 280 936 and the Czech Utility Model No. 5634 or the published Czech Application No. PV 1991-3174).

Besides flavonolignans the milk thistle fruit contains other biologically significant substances as oil containing multiply unsaturated fatty acids, in particular linoleic acid. This oil is used in the cosmetic industry (see the Czech Patent No. CZ 294 004), in pharmaceutical industry (see e.g. the Russian Patent No. RU 2 099 076), in the sphere of animal feeding (see e.g. the Czech Patent No. CZ 285 745), and also as the raw material for the production of biologically active derivatives of linoleic acid (see e.g. the Czech Application No. PV 2007-25). According to the state of the art general use of linoleic acid in combination with other natural substances is very frequent in human food supplements as disclosed in the US Patent No. 6 576 252.

While treated milk thistle fruit plays an important role in the human as well as veterinary sphere as the final product for direct consumption (see e.g. the published International Application No. WO 2007/020382), it is still primarily used as the raw material for the production of flavonolignan concentrates (sylimarin) or for extraction of oil respectively. Pressure on price reduction of the final product is reflected in the need to produce the raw material with as high content of active substances as possible. This can be achieved by improvement of the particular plant species or interventions into its genetic fund (see e.g. the German Patent No. DE 4 231 634), and/or by treatment of the milk thistle fruit having the standard content of active substances. In the state of the art the milk thistle fruit is most frequently extracted after simple disintegration (grinding) (see e.g. the published international application No. WO 2004/039386, Belgian Patent No. BE 1 000 468 or Korean Patent No. KR 8 902 755) or after prior pressing (see e.g. the US Patent No. 4 368 195), when the oil present in the fruit is removed (see e.g. the Czech Patent No. CZ 295 611 or the published Czech application No. PV 2005-8). The reason for this situation is the fact that division of the milk thistle fruit with subsequent separation of the inner and outer layers places high technological difficult and is widely reflected into the price of the raw material. Plain increase of the concentration of flavonolignans, however including the present ballast substances, cannot compensate the higher price of the raw material, even with regard to the fact of productivity increase of individual pieces of production equipment necessary for the production of the final product. The main problem in this case remains the necessity to refine the primarily obtained extracts in a complicated way with the aim to remove the above mentioned ballast substances with them. A general complication within the refining of extract is the necessity to use special equipment and auxiliary substances, especially solvents, also from the group of chlorinated or aromatic solvents that represent a serious risk from the point of view of the environment and residues in the final products. These problems are discussed in some of the above mentioned patents or e.g. the Rumanian Patents No. RO 74 800, RO 84 556 and the GB Patent No. 2 084 569.

The application of the approach when ballast substances would be removed with extraction methods before the extraction of the entire active substances cannot be efficiently used in principle with regard to the fact that the physical-chemical properties of many of the ballast substances are very similar to those of the active substances and losses of these substances would be the result. In spite of this according to the state of the art pre-extraction of the milk thistle fruit is used (see e.g. the German Patent No. DE 10 016 449), but having the aim to only remove lipophilic ballast substances from the fruit, especially oils. This approach does not sufficiently solve the problem of all ballast substances and in addition it introduces an additional technological step in the production - pre-extraction of the raw material which is considerably technologically demanding in the case of using environment-friendly extraction methods, e.g. with the use of carbon dioxide in the supercritical status (see e.g. the Chinese Patent CN 1 463 970).

### Disclosure of Invention

The above mentioned shortcomings are overcome with the mechanically treated milk thistle fruit and the method of its treatment according to present invention, which provides high-quality raw materials for the production of the flavonolignan concentrate (silymarin), milk thistle oil and divided fractions of the fruit suitable for direct use both in the human and veterinary area.

Processing of the milk thistle fruit with its mechanical treatment provides a lot of possibilities how to prepare concentrates of active substances obtainable from this material. The substance of the invention consists in obtaining the mechanically treated of milk thistle fruit while finding a method comprising a combination of technological operations where the milk thistle fruit is treated by the action of mechanical forces in such a way that concentration of individual groups of active substances in the resulting materials can be achieved and undesired and ballast substances are removed.

From the point of view of active substances there are mainly two basic groups of compounds: Flavonolignans (substances from the flavonoid group) and fatty oils (substances from the groups of triglycerides of fatty acids). According to this invention mainly the increased concentration of flavonolignans represents the principal goal in the combination of mechanical treatment of the milk thistle fruit. According to this invention this goal is achieved by division of the milk thistle fruit when the undesired surface layer is removed from the fruit. This way the bare fruit is obtained containing the inner parts of the fruit, i.e. the endosperm, together with parts of the outer fruit coats (husk, bran). The results of performed semi-operational and subsequent operational tests have shown that flavonolignans are primarily contained in this material and that partial or complete separation of the surface layers from the inner layers of the fruit leads to their increased concentration in comparison to the average content in the whole fruit. With further mechanical division of the milk thistle fruit treated this way the remaining inner layers of the fruit can be obtained, which however, depending on the used separation technology, may contain a lower or higher quantity of the outer fruit coats. This material is mainly rich in polysaccharides (starch), fibre and fatty oils. The other product is the entire fruit coats containing a high content of flavonolignans and low content of oils.

Flavonolignan concentrates obtained with the above mentioned alternatives of the method of mechanical treatment of the milk thistle fruit according to the present invention can be used to produce the highly concentrated flavonolignan complex called silymarin with common extraction methods with the use of organic solvents as the extraction agent. However, these concentrates can be preferably further treated e.g. by pressing, pelletizing or granulating to achieve the physical-mechanical characteristics mentioned below, which are necessary for desirable operation of most commonly used extraction technologies, i.e.:
1) Creation of a granulated form of the particular material whose compressive strength in the dry condition is higher than 0.2 MPa;
2) Control of distribution of granulate particles particularly in the range from 0.5 m to 12.0 mm;
3) Control of the content of flavonolignans to a pre-determined concentration;
4) Control of the content of fatty oils.

To obtain flavonolignan concentrates with the above mentioned characteristics several technological alternatives were tried according to the invention. After obtaining of the milk thistle fruit whose drying loss must be below 12 % by weight, preferably below 9.0 % by weight with optionally additional drying (step a) mechanical impurities are separated if their content exceeds 2.0 % by weight related to the total weight of the fruit (step b). Then, the milk thistle fruit is subject to separation of the outer layer (step c). This separation is achieved by the action of mechanical forces onto the fruit in a machine (mill) where the fruit gets in contact with grinding plates the movement of which gradually removes, e.g. abrades the outer layer of the fruit. In this step in the case of fruits where the inner parts of the fruit tightly fit the outer fruit coats just the outer layers are ground off. In the case of fruits where the inner parts of the fruit are partly or completely separated from the layers of the outer fruit coats due to drying after grinding of the outer layers the fruit coats are often peeled off and the remaining inner layers (endosperm) are completely exposed. The material divided this way is preferably separated directly in the equipment after leaving of the grinding plates with the use of sieves with a suitable mesh size while the abraded outer layers in the form of fine dust pass through the sieves and the inner parts of the fruit remain on the sieves. This way both the types of material with different contents of flavonolignans as well as other substances, especially fatty oils, are separated. The whole process is preferably carried out at the room temperature. The primary control of the grinding rate is based on the setting of the weight flow of the incoming material to the equipment by the corresponding regulation element of the equipment. The actually separated quantity of the ground-off material can be controlled with the mesh size of the output sieves in the equipment. Practical semi-production and commercial-scale experiments according to the invention showed that the optimum weight proportion of the ground-off outer layers to the inner parts of the fruit was 1 : 9 to 2 : 8 depending on the required content of flavonolignans in individual output materials. It is obvious for the skill man in the art will that separation of the inner layers of the milk thistle fruit can also be beneficially achieved by pealing and/or tumbling and/or extrusion. It is also obvious that individual above mentioned methods can be mutually combined.

The ground-off outer layers of the milk thistle fruit obtained this way have the defined maximum size of particles as a result of passing through the sieve and they only contain a small proportion of flavonolignans. However, they are rich in substances forming the protective layer of the fruit, in particular natural wax, phytosterols, polymeric terpenic substances and higher aliphatic hydrocarbons. For this reason this material is suitable for their isolation, namely in the direct use mode in all types of extraction technologies where finely ground materials are required, e.g. in charge mixed extractors. On the other hand, for extractions carried out in percolators (percolator sets) or continuous equipment of the carousel extractor types the structure of extracted material is subject to different requirements as mentioned above. For these reasons the ground-off outer layers of the milk thistle fruit may be further processed by the action of mechanical forces in such a way to ensure mainly their compaction. These operations may be executed in granulators, pelletizing machines or presses. Especially the use of presses appears to be very advantageous as within the application of this technology you can precisely control the pressing proportions by setting the machine pressure and at the same time you can press the defined quantity of fatty oil.

According to another aspect of the invention the inner parts of the milk thistle fruit can be further preferably mechanically treated. The primary goal of this further treatment is to control the content of flavonolignans and fatty oil in the resulting material and to increase extractability of these substances within subsequent extraction or to increase their biological availability within the direct use in food or feeding applications. The secondary goal according to the invention is to obtain a material with optimum physical characteristics for a particular purpose of use (mechanical strength, size of particles, etc.). For these purposes it is advantageous to process the inner parts of the fruit by pressing again (step d1). If a lower content of flavonolignans should be achieved than pressing of the particular material of the inner parts of the milk thistle fruit would provide, according to the present invention before the pressing this material can be preferably homogenized with the whole milk thistle fruit or its part so the resulting average value of the content of flavonolignans in the resulting product after the pressing may be maintained on a pre-defined level. If a higher content of flavonolignans should be achieved than pressing of the particular material of the inner parts of the milk thistle fruit would provide, the fraction of free fruit coats with a high content of flavonolignans can be preferably mechanically separated from the fraction of the remaining inner layers of the fruit where the content is flavonolignans is low before the pressing, e.g. on sieves and/or gravity tables and/or with the use of an optical sorter (step d2). The fraction of fruit coats can be then directly pressed to a material with a very high content of flavonolignans (step e3) or before pressing it can be homogenized together with inner parts of the milk thistle fruit and by pressing this blended material you can obtain the resulting product with the defined content of flavonolignans higher than would only correspond to pressed inner parts of the fruit. The proportion of the fraction of free fruit coats in the inner parts of the fruit before sorting can be increased by additional mechanical disintegration of the outer parts of the fruit, e.g. during the second passage through the machine used for the grinding of the whole fruit. Analogously, you can use the separated fraction of the remaining inner layers of the fruit with a low content of flavonolignans for homogenization with the inner parts of the milk thistle fruit to achieve a reduction of the content of flavonolignans to a pre-defined value.

For the entire pressing you can beneficially use screw presses, especially with multiple thread worm. The compression pressures typically vary in the range from 30.0 MPa to 70.0 MPa and the content of residual fatty oil in the pressed material (oil cake) in between 5 % by weight and 11 % by weight.

A typical product after pressing of the above mentioned materials or their mixtures obtained by the mechanical treatment of the milk thistle fruit in screw presses are flakes of flat, slightly cylindrically rolled irregular shapes with the typical dimensions from 1 mm to 80 mm. The thickness of the flakes results from the size of the press slot and varies in the range from 1 to 8 mm. Material with this distribution of sizes and shape of particles is suitable for direct use in some types of extractors, especially continuous extractors of the carousel type. In these devices counter-flow contact of the extraction agent with the extracted material occurs, which results in the highest extraction efficiency. For this reason, even larger material agglomerates can be extracted while maintaining a high extraction yield. These larger agglomerates are desirable in particular for maintaining hydro-dynamical characteristics of the layer of the extracted material in the extractor in such a way that the extraction agent can stream over the layer of the extracted material with its own weight without the necessity to pressurize the system and at the same time that the slots of the extractor bottom designed to drain the liquid extract should not get clogged by fine fractions of the extracted material. However, for some types of extractors, typically for a percolator set, it is suitable to treat the primarily obtained product after pressing by crushing and sieving. This operation can be performed in one step in a rotational crusher equipped with a system of sieves with different mesh sizes at the output. The incoming material is separated by the rotational movement of a knife into smaller particles that gradually pass through the system of sieves with different mesh sizes depending on their size. Individual material fractions are collected by the required distribution of particle sizes. Large particles may be returned back to the beginning of the crushing process; the smallest dust particles may be returned to the preceding pressing process. The whole operation of crushing and sieving is carried out at the room temperature. The resulting material has the defined distribution of sizes of particles whose shape becomes spatially symmetrical due to crushing and sieving. These parameters are especially important for the particular extraction technology in a percolator set since they determine the pressure proportions within the entire extraction and the extraction speed with a particular geometry of the extractor structure and depending on the used organic solvent. Further, material with spherized particles according to the invention shows a much better stability during the storage and transport of material, when vibrations do not cause substantial secondary crushing of material particles or their segregation in layers by the particle size. Also, the rheological behaviour of loose material during its transport in transport paths is optimum and allows using of both screw and pneumatic transport system. Finally - crushed and sieved material can be efficiently packed due to its high powder density and transported with maximum reductions of storage and transport costs.

According to this invention the use of the isolated fraction of the remaining inner layers of the milk thistle fruit with a lower average content of flavonolignans as compared to the average content in the whole fruit has also been verified. This material can be used in applications where not only the effects of flavonolignans, but also the effects of the other substances contained in this material should be used. The remaining inner layers of the milk thistle fruit are rich in polysaccharides, proteins, fibre, fatty oils and phytosterols. With regard to the content of these components this material is suitable for use as a food supplement. The use in this sphere has been limited mainly by substances contained in the outer fruit coats and surface layers of the fruit that caused its bitter taste (bitter substances, lignan polymers, flavonoids, products of partial oxidation of natural substances, plant wax, etc.); however, according to this invention after abrading of the outer layers and removing of the fruit coats most of these characteristics causing unpleasant taste are lost and so the remaining inner layers of the fruit can be used for direct consumption, for addition to bakery (whole-grain bakery), cereals (cereal bars), healthy-diet products (cornflakes, mixtures, muesli), etc.

In most cases, however, the use of further unprocessed residues of the inner layers of the milk thistle fruit is limited by the relatively high content of fatty oil. The oil content in this material, depending on the degree of removal of the outer layers, varies between 17 % by weight and 35 % by weight related to the total weight of the milk thistle. Although this oil contains essential fatty acids, in particular linoleic acid, its excessive content could cause diet problems during consumption. According to this invention processing of the remaining inner layers of the fruit, primarily obtained after separation of the ground-off outer layers, by subsequent pressing is preferable and ideal (step e2). With regard to a reduced content of the hard parts of the fruit after grinding under common compression pressures a content of residual fatty oil below 7.0 % by weight related to the total weight of the milk thistle can be achieved. The material treated this way can be further crushed and sieved once again as described above and with the same advantages. In addition, during material pressing due to the influence of applied pressures and mechanical pressures statistically significant disturbance of the cell walls occurs, which increases biological availability of all substances contained in the cells. This fact is mainly important with regard to biological availability of flavonolignans from the particular material.

According to the invention outside the food industry untreated or pressed (step e2) and optionally subsequently crushed and sieved (step f2) residual inner layers of milk thistle fruits can be used in the sphere of feeding supplements for farm animals and pets. The improvement of taste characteristics allows addition of this material to feeding mixtures not only for cattle, but also for pigs, poultry, horses, cats, dogs and fish, where the use of milk thistle has been limited by its bitter taste. This material can be used for the preparation of all common feeding mixtures or it can be added directly to feeding charges of animals.

With the use of suitable technologies and extraction agents pressed optionally crushed and sieved inner layers of milk thistle fruits can also be used as the raw material for isolation of flavonolignans.

According to the invention the fatty oil obtained by pressing of the inner parts of the milk thistle fruit (step d1) has an excellent quality. If a cold pressing process is used, the virgin oil produced this way keeps a high nutritious value. The high quality of oil obtained this way as compared to oil obtained by pressing of whole fruits of milk thistle mainly results from the absence of the outer layers of the fruit that release lipophylic substance into the oil, which generally reduce the quality of oil. In particular this is the case of partly oxidized oil, free fatty acids and partly hydrolyzed fats. Presence of these substances increases the peroxide number and the oil acidity number. The uppermost layer of the fruit (called cutin) protects it from the external environment and in particular contains natural waxes. If this layer is removed, the waxes do not get into the oil and the quality of the oil gets higher since its turbidity temperature, i.e. temperature at which the fixed fraction is separated from oil is reduced. This fact is important mainly with regard to the use of milk thistle oil as an alternative fuel for the production of bio-diesel (fatty acid esters with lower aliphatic alcohols) or as a direct bio-fuel for diesel engines or sources of heat. Further, due to the abrasion of the outer layers of the fruit such substances are beneficially removed that decrease the quality of oil from the sensoric point of view, especially as regard colour, smell and taste. Oil obtained by pressing of inner parts of the milk thistle fruit, depending of the particular purpose of the intended use either does not require any or requires just minimum refining to achieve its desired quality parameters. Oil produced this way can be used a food, a food supplement (source of omega-6 fatty acids), a feeding supplement, for cosmetic purposes, for the production of treatment cosmetics ("cosmeneutics"), for the production of semi-synthetic food supplements (e.g. conjugated fatty acids and their derivatives), or as a natural source of fatty acids for the production of semi-synthetic derivatives of these substances (especially esters) with applications in pharmaceutical, cosmetic, food, engineering industry, etc.

Preferably, together with the oil obtained by pressing of the inner parts of the milk thistle fruit (step d1) oil obtained by pressing of the separated fraction of the remaining inner layers of this fruit (step e2) also shows the above mentioned qualitative characteristics. However, in some indicators (colour, acidity number) its quality is even higher than in the case of oil pressed from the inner parts of the fruit, which results from the absence of fruit coats that may to a lower extent contain substances that negatively influence the quality of oil. Thus, oil obtained by pressing of the residual inner layers of the fruit is suitable for the most demanding applications in the cosmetic, food and pharmaceutical industry as mentioned above.

All the technology of the mechanical processing of the milk thistle fruit and the layout of individual assemblies according to the technology has been preferably designed in such a way to use gravity technologies to the maximum possible extent, which means that gravity is used for transport within the particular technological assembly. This method considerably reduces production costs and minimizes impacts of the production on the environment.

Another important feature of the invention is the use of wasteless technologies. This feature results from utilization of all the primary and secondary products of the entire processing of the milk thistle fruit with the above mentioned methods.

The production of products according to the invention is also in full compliances with requirements for environment protection. The production consumes minimum amounts of electricity, all the processes are low-heat and only require the supply of "clean" electricity. Also, the disposal of used auxiliary materials from the production (filtration partitions, adsorbents) is fully environment-friendly since these materials themselves, including the captured substances are fully bio-degradable and can be disposed of e.g. by composting or adding to reactors of wastewater treatment plants as sources of required nutrients. In general, the treatment of the milk thistle fruit complies with the aim to use the maximum potential of raw materials that can be obtained from fully renewable natural resources.

With regard to the fact that materials obtained by mechanical processing of the milk thistle fruit according to the invention are used for the needs of isolation of silymarin as and active pharmaceutical substance (API) the technologies are designed for operation under the conditions of Good Manufacturing Practice (GMP). This high standard of quality management of processes and products that is required for the production of API is also applied within the system of technologies to all the other secondary products that do not belong to the sphere of pharmaceutical production. Under these conditions these materials are also produced on the highest current level of knowledge of processes from the quality point of view and thus they can comply with all the legislative requirements for applications in the food and feeding industry.

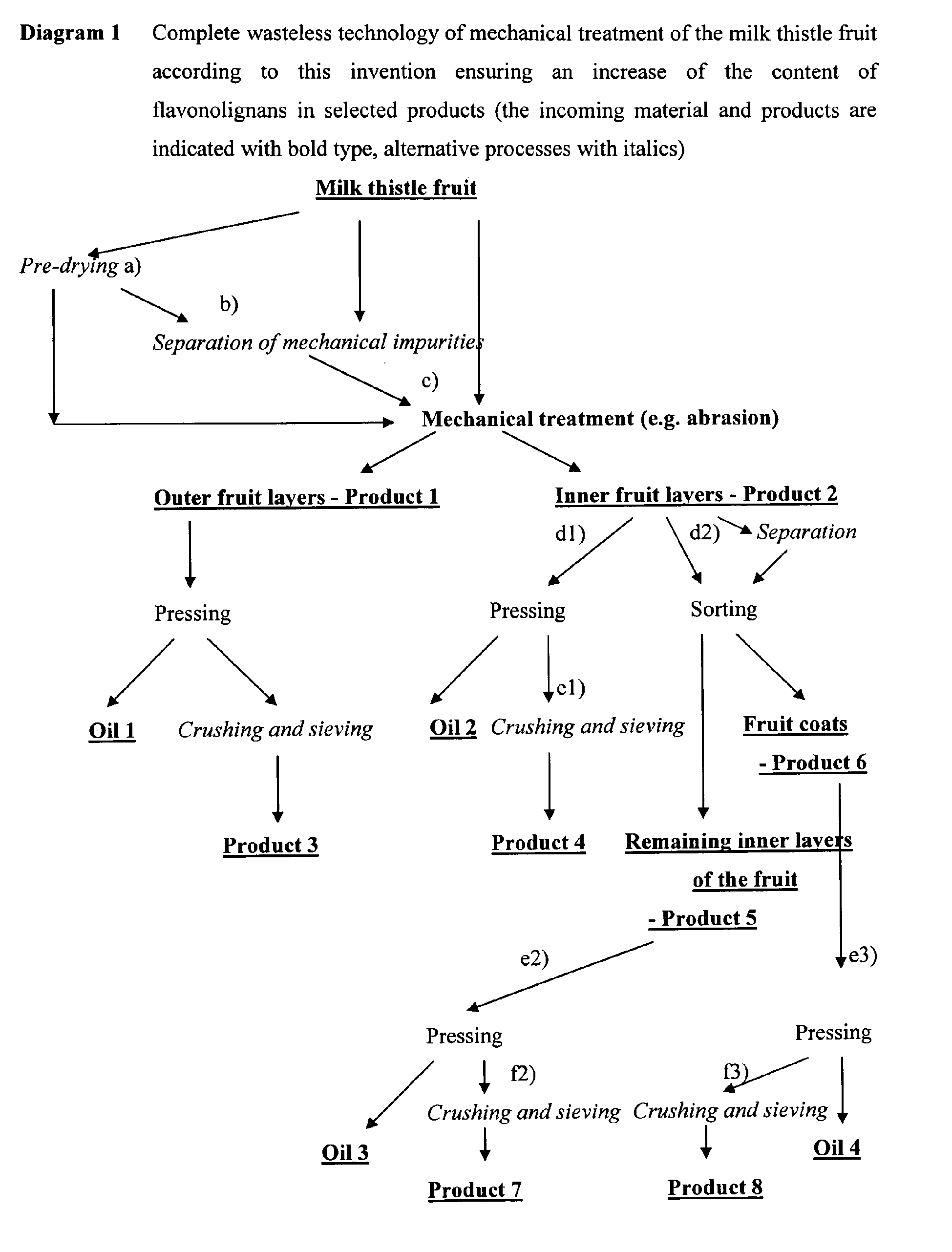

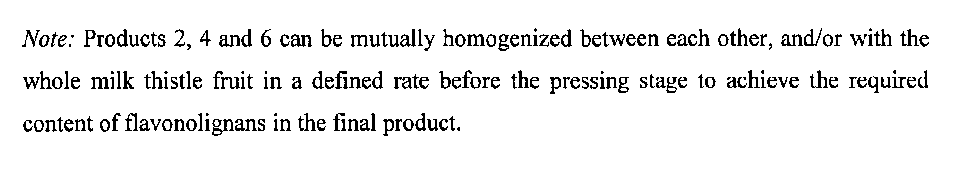

### Examples

### Example 1 Treatment of the milk thistle fruit by abrading and subsequent pressing (commercial-scale experiment)

A provided charge (10200 kg) of the milk thistle fruit (drying loss 7.05 % by weight, content of flavonolignans 2.96 % by weight, content of foreign admixtures 0.32 % by weight) was subject to abrasion in a peeling machine (type: "Ekonos" 3.22, manufacturer: TMS Group, a.s., Pardubice) at the weight flow of 800 kg.h⁻¹. The machine was equipped at the output with a sieve with the mesh size of 1.2 x 20 mm. 1240 kg of ground-off outer layers of the fruit (Product 1) and 8940 kg of bare fruit (Product 2) were obtained. Products 1 and 2 were further processed:
a) 1200 kg of product 1 were pressed in a multiple-threaded worm press (type: "Shark" PL 200 LCPV7, manufacturer: Farmet, spol. s r. o, 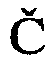 eská Skalice) at the pressure of 70 MPa and 1078 kg of compressed Product 3 and 72 kg of Oil 1 were obtained.
b) 7800 kg of Product 2 were pressed in a multiple-threaded worm press of the above mentioned type at the pressure of 45 MPa and 6621 kg of compressed Product 4 and 1050 kg of Oil 2 were obtained.

All the Products 1 to 4 were sampled for the corresponding analyses. The results of the analyses are summarized in Table 1.

**Table 1 Results of analyses of individual products of processing of the milk thistle fruit by abrading and subsequent pressing**

| | | | |
|---|---|---|---|
| Product No. | Drying loss in % by weight | Oil content in % by weight | Content of flavonolignans in % by weight in the dried matter |
| 1 | 6.16 | 11.8 | 1.97 |
| 2 | 5.66 | 20.1 | 3.06 |
| 3 | 4.21 | 4.6 | 3.02 |
| 4 | 5.86 | 7.7 | 4.26 |

### Example 2 Treatment of the milk thistle fruit by abrading and pressing or abrading, separating, partial sorting and pressing (commercial-scale experiment)

A provided charge (2000 kg) of the milk thistle fruit (drying loss 6.48 % by weight, content of flavonolignans 4.36 % by weight, content of foreign admixtures 0.25 % by weight) was subject to abrading in the peeling machine as in Example 1 with the weight flow of 800 kg.h⁻¹. The machine was equipped at the output with a sieve with the mesh size of 1.2 x 20 mm. 226 kg of ground-off outer layers of the fruit (Product 1) and 1734 kg of the bare fruit (Product 2 were obtained. Product 2 was processed by individual parts:
a) 786 kg of Product 2 were pressed in the multiple-threaded worm press as mentioned in Example 1 under the pressure of 48 MPa and 635 kg of compressed Product 4 and 106 kg of Oil 2 were obtained.
b) 948 kg of Product 2 were separated in the peeling machine of the above mentioned type at the weight flow of 400 kg.h⁻¹. The machine was equipped at the output with a sieve with the mesh size of 1.2 x 20 mm, which serves as a partial sorting element in this case. 368 kg of sieved material (product 6) and 560 kg of material remaining on the sieve (Product 5) were obtained. Then, 100 kg of Product 6 were pressed in the multiple-threaded worm press of the above mentioned type at the pressure of 65 MPa and 91 of compressed Product 8 and 4 kg of Oil 4 were obtained. In the next step 360 kg of Product 5 were pressed in the multiple-threaded worm press of the above mentioned type at the pressure of 47 MPa and 262 kg of compressed Product 7 and 68 kg of Oil 3 were obtained.

All the Products 1, 2 and 4 to 8 4 were sampled for the corresponding analyses. The results of the analyses are summarized in Table 2.

**Table 2 Results of analyses of individual products of processing of the milk thistle fruit by abrading and pressing, or abrading, separating, sorting and pressing**

| Product No. | Drying loss in % by weight | Oil content in % by weight | Content of flavonolignans in % by weight in the dried matter |
|---|---|---|---|
| 1 | 6.20 | 11.0 | 2.50 |
| 2 | 5.82 | 19.6 | 2.55 |
| 4 | 5.97 | 6.8 | 3.98 |
| 5 | 5.28 | 24.4 | 2.01 |
| 6 | 6.07 | 9.9 | 2.87 |
| 7 | 6.25 | 7.1 | 3.52 |
| 8 | 5.04 | 5.8 | 4.97 |

### Example 3 Treatment of exposed, separated and party sorted milk thistle fruit by complete sorting and pressing (semi-production experiment)

A provided charge (200 kg) of exposed, separated and partly sorted milk thistle fruit (Product 5 from Example 2) was finally sorted in a gravity sorter (type: HEID - GA 110L, manufacturer Cimbria Heid, GmbH, Austria) at the material flow rate of 800 kg.h⁻¹. The sorting provided the fraction of fruit coats with the weight of 20 kg (Product 6) and the fraction of the inner layers of the fruit with the weight of 176 kg (Product 5). Then 20 kg of Product 6 were pressed in the multiple-threaded worm press as mentioned in Example 1 at the pressure of 70 MPs and 18 kg of compressed Product 8 and 0.8 kg of Oil 4 were obtained.
Similarly, 176 kg of Product 5 were pressed in the multiple-threaded worm press of the above mentioned type at the pressure of 40 MPa and 135 kg of compressed Product 7 and 37 kg of Oil 3 were obtained.

All the Products 5 to 8 were sampled for the corresponding analyses. The results of the analyses are summarized in Table 3.

**Table 3 Results of analyses of individual products of processing of exposed, separated and partly sorted milk thistle fruit by complete sorting and pressing**

| Product No. | Drying loss in % by weight | Oil content in % by weight | Content of flavonolignans in % by weight in the dried matter |
|---|---|---|---|
| 5 | 5.63 | 26.1 | 2.37 |
| 6 | 6.18 | 10.2 | 6.12 |
| 7 | 6.34 | 5.9 | 3.61 |
| 8 | 6.02 | 6.7 | 9.03 |

### Example 4 Treatment of exposed and pressed milk thistle fruit by crushing and sieving (commercial-scale experiment)

A provided charge (6600 kg) of pressed exposed milk thistle fruit (Product 4 from Example 1) was crushed and sieved in a machine (type: PS-12, manufacturer Haiva, spol. s r. o., Tasovice) equipped at the output with a sieve with the mesh size of 8 mm. The material feed speed through the crusher was 6000 kg.h⁻¹. 6580 kg of crushed and sieved Product 4 were obtained altogether.

A sample of the crushed and sieved Product 4 was obtained for a sieve analysis. The results of the analysis are summarized in Table 4.

**Table 4 Results of the sieve analysis of the crushed and sieved product obtained by sieving of exposed milk thistle fruit**

| Product No. | Particle size distribution in % by weight | | | |
|---|---|---|---|---|
| | Below 0.5 mm | 0.5 mm - 2.0 mm | 2.0 mm - 4.0 mm | 4.0 mm - 8.0 mm |
| 4 | 5 | 28 | 51 | 16 |

### Example 5 Treatment of the exposed milk thistle fruit by separation, sorting, pressing, crushing and sieving (commercial-scale experiment)

A provided charge (1000 kg) of the milk thistle fruit (Product 2 from Example 1) was processed by separation in a peeling machine (type: HN-1000, manufacturer: Seed-Imex, Co. Ltd., Hungary) at the material flow rate of 400 kg.h⁻¹. 478 kg of material containing mainly fruit coats and 506 kg of material containing mainly inner layers of the fruit were obtained. The material containing mainly fruit coats was subject to finally sorting in a gravity sorter as mentioned in Example 3 at the material flow rate of 800 kg.h⁻¹. The sorting produced a pure fraction of fruit coats with the weight of 421 kg (Product 6) and a fraction containing mainly inner layers of the fruit with the weight of 52 kg (Product 5). Then, Product 6 was pressed in the multiple-threaded worm press as mentioned in Example 1 at the pressure of 67 MPa and 403 kg of compressed product 8 and 16 kg of Oil 4 were obtained. Product 8 was then crushed and sieved in the machine mentioned in Example 4, equipped with a sieve with the mesh size of 12 mm at the output. The material feed rate through the crusher was 3000 kg.h⁻¹. 396 kg of crushed and sieved Product 8 were obtained altogether

All the Products 5, 6 and 8 were sampled for the corresponding analyses. The results of the analyses are summarized in Tables 5 and 6.

**Table 5 Results of analyses of individual products of processing of the exposed milk thistle fruit by separation, sorting, pressing, crushing and sieving**

| Product No. | Drying loss in % by weight | Oil content in % by weight | Content of flavonolignans in % by weight in the dried matter |
|---|---|---|---|
| 5 | 6.34 | 25.6 | 2.27 |
| 6 | 5.92 | 9.3 | 6.51 |
| 8 | 5.85 | 5.7 | 9.79 |

**Table 6 Results of the sieve analysis of the crushed and sieved product obtained by pressing of sorted fruit coats of milk thistle**

| Product No. | Particle size distribution in % by weight | | | | |
|---|---|---|---|---|---|
| | Below 0.5 mm | 0.5 mm - - 2.0 mm | 2.0 mm - - 4.0 mm | 4.0 mm - - 8.0 mm | 8.0 mm - - 12.0 mm |
| 8 | 4 | 22 | 45 | 19 | 10 |

### Industrial Applicability

Mechanically treated exposed milk thistle fruit with concentrated active component - flavonolignans, with the defined distribution of the size of particles and defined low quantity of the fatty component is suitable for subsequent extraction processing with the aim to isolate the pure flavonolignan fraction. The absence of the surface layers containing undesired and ballast substances in the extracted material contributes to an increase of flavonolignans in the dry matter of the primary extracts and substantially facilitates their subsequent refining leading to a highly concentrated flavonolignan fraction. These factors in connection with the achieved high concentration of flavonolignans in the incoming material considerably improve the economy of production of the concentrated flavonolignan fraction (so-called silymarin) as a result of the possibility of simplification of the production technological procedures, increase of productivity of the production equipment and reduction of costs of auxiliary raw materials, energy and workforce.

The inner parts of the milk thistle fruit as we as materials obtained by its further specific mechanical processing lose most of the undesired sensoric characteristics and thus they can be directly used in the food and feeding industry as sources of flavonolignans and other active constituents with high biological availability.

A secondary product of processing of the inner parts of the milk thistle fruit is vegetable oil, which with regard to the conditions of its production contains maximally preserved desirable natural nutritious components. With regard to the high quality of oil obtained this way within most of its applications, in the food, feeding, cosmetic and pharmaceutical industries there is no need to refine it any further.

## Claims

1. A mechanically treated milk thistle fruit suitable particularly for extraction of pharmaceutically active substances, **characterized in that** it contains 80 to 90 % of the original weight of the untreated fruit without the outer layers of the fruit due to which it is enriched by the inner parts of the milk thistle fruit having an increased content of flavonolignans and oil as compared to their average content in the untreated fruit.

2. The mechanically treated fruit according to claim 1, **characterized in that** it contains 86 to 88 % by weight of the original untreated fruit.

3. The mechanically treated fruit according to claim 1 or 2, **characterized in that** the inner parts of the fruit contain from 2.5 to 4.5 % by weight of flavonolignans and from 16 to 26 % by weight of oil of the total weight of the fruit.

4. The mechanically treated fruit according to claims 1 to 3, **characterized in that** the inner parts of the fruit comprise the residual inner layers of the fruit and fruit coats of the milk thistle.

5. The mechanically treated fruit according to claim 4, **characterized in that** it consists of compacted, preferably compressed optionally mechanically treated inner parts of the milk thistle fruit having the content up to 5.5 % by weight of flavonolignans in the fruit.

6. The mechanically treated fruit according to claim 4, **characterized in that** it consists of the remaining inner layers of the milk thistle fruit after sorting of the inner parts of the fruit having the oil content up to 27 % by weight and the content of flavonolignans of max. 2.7 % by weight.

7. The mechanically treated fruit according to claim 6, **characterized in that** it consists of compacted, preferably compressed remaining inner layers of the milk thistle fruit having the oil content of max. 7.2 % and the content of flavonolignans up to 3.7 %.

8. The mechanically treated fruit according to claim 4, **characterized in that** it consists of fruit coats of milk thistle after sorting of the inner parts of the fruit with the oil content up to 11 % and the content of flavonolignans of max. 6.6 % by weight.

9. The mechanically treated fruit according to claim 8, **characterized in that** it consists of compacted, preferably compressed, optionally subsequently mechanically treated fruit coats of milk thistle having the oil content of max. 6.8 % and the content of flavonolignans up to 9.8 % by weight.

10. The mechanically treated fruit according to claim 1 to 9, **characterized in that** it consists of a compacted mixture of the inner parts of the milk thistle fruit and the mechanically untreated fruit or parts of the milk thistle fruit.

11. A method of mechanical treatment of the milk thistle fruit according to claim 1 that it comprises the following steps:
a) pre-drying of the milk thistle fruit, if necessary;
b) separating of mechanical impurities, if necessary;
**characterized in that**
c) 10 to 20 % by weight of the outer layer of the milk thistle fruit is further mechanically removed to increase the content of flavonolignans and oil in the inner part of the milk thistle fruit.

12. The method according to claim 11, **characterized in that** 12 to 14 % by weight of the outer layer of the milk thistle fruit is removed.

13. The method according to claim 11 or 12, **characterized in that** the mechanical removal comprises abrading and/or peeling, and/or grinding-off, and/or extrusion.

14. The method according to any preceding claims 11 to 13, **characterized in that** step c) is carried out at the ambient temperature, max. 35 °C.

15. The method according to any preceding claims 11 to 14, **characterized in that** after the mechanical separation of the outer layers of the fruit from the inner parts in step c) the fractions of the inner parts of the fruit are subject to step d1) compaction, preferably compression, optionally with subsequent crushing and sieving in step e1) or, in step d2) they are optionally mechanically separated and then mechanically sorted, preferably in a gravity way, and/or by sieving into the inner layers of the fruit and fruit coats.

16. The method according to claim 15, **characterized in that** the remaining inner layers of the fruit are subjected to the next step e2) of the mechanical treatment, which is preferably pressing.

17. The method according to claim 16, **characterized in that** the remaining inner layers of the fruit are subsequently subjected to step f2) of the mechanical treatment, which is preferably crushing with optionally sieving.

18. The method according to claim 15, **characterized in that** the fruit coats of milk thistle are subjected to the next step e3) of the mechanical treatment, which is preferably pressing.

19. The method according to claim 18, **characterized in that** the compressed fruit coats are subsequently subjected to step f3) of the mechanical treatment, which is preferably crushing and then optionally sieving.

20. Use of the mechanically treated milk thistle fruit according to any of the claims 1 to 10 in the feeding, food, cosmetic or pharmaceutical industry.
